# NEUE EUROPÄISCHE PATENTSCHRIFT

(11) **EP 0 220 714 B2**
(45) Veröffentlichungstag und Bekanntmachung des Hinweises auf die Entscheidung über den Einspruch: **29.11.1995**
(45) Hinweis auf die Patenterteilung: 18.03.1992
(21) Anmeldenummer: 86114951.6
(22) Anmeldetag: 28.10.1986
(51) Int. Cl.: C12N 15/54, C12N 9/10

(54) **DNA-Fragment mit dem Cyclodextrin-Glycosyl-Transferase-Strukturgen, Expressionsvektor, Mikroorganismen zur Expression und Herstellungsverfahren**
DNA fragment containing the cyclodextrin-glycosyl-transferase gene, expression vector, microorganisms for expression and preparation process
Fragment d'ADN contenant le gène structurel de la cyclodextrine-glycosyl-transférase, vecteur d'expression, micro-organismes pour l'expression et procédé de préparation

(30) Priorität: 29.10.1985 DE 3538433
(43) Veröffentlichungstag der Anmeldung: 06.05.1987
(73) Patentinhaber: Consortium für elektrochemische Industrie GmbH, D-81379 München (DE)
(72) Erfinder: Böck, August, Prof. Dr., D-8085 Geltendorf (DE); Binder, Florian, D-8000 München 80 (DE); Müller, Frank, Dir. Dr., D-8011 Höhenkirchen (DE)

(56) Entgegenhaltungen:
- FR-A- 2 213 340
- FR-A- 2 574 081
- CHEMICAL ABSTRACTS, Band 105, Nr. 21, November 1986, Seite 595, Zusammenfassung Nr. 189477m, Columbus, Ohio, US
- JOURNAL OF BACTERIOLOGY, Band 166, Nr. 3, Juni 1986, Seiten 1118-1122, The American Society for Microbiology, Washington, D.C., US; T. TAKANO et al.: "Molecular cloning, DNA nucleotide sequencing, and expression in Bacillus subtilis cells of the Bacillus macerans cyclodextrin glucanotransferase gene"
- CHEMICAL ABSTRACTS, Band 107, Nr. 9, 31. August 1987, Seite 193, Zusammenfassung Nr. 72089m, Columbus, Ohio, US
- PATENT ABSTRACTS OF JAPAN, Band 10, Nr. 321 (C-382)[2377], 31. Oktober 1986
- R.W. OLD et al.: "Principles of gene manipulation", 3. Auflage, 1985, Seiten 138-140, Blackwell Scientific Publications, Oxford, GB
- STARCH-STÄRKE, Band 33, Nr. 8, August 1981, Seiten 281-283, Verlag Chemie GmbH, Weinheim, DE; E. LASZLO et al.: "Purification of cyclodextrin-glycosyltransferase enzyme by affinity chromatography"
- ARCH. MICROBIOL., Band 111, 1977, Seiten 271-282, Springer Verlag, Berlin, DE; H. BENDER: "Cyclodextrin-Glucanotransferase von Klebsiella pneumoniae"
- T. MANIATIS et al.: "Molecular cloning: A laboratory manual", 1982, Seiten 295-306, Cold Spring Harbor Laboratory, NY, US
- J. Biol. Chem. 258, 1007-1013 (1983), Takkinen et al.
- J. Bacteriol. 163, 401-406 (1985), Nakajima et al.
- Arch. Microbiol. 111, 271-282 (1977), H. Bender
- I. Int. Symp. on Cyclodextrins, Budapest (1981), Landert et al., A Photometric Test for Cyclisation Activity of Cyclodextrin-Glycosyltransferases
- Agr. Biol. Chem. 38, 12, 2413-2417 (1974), Kitahata et al.
- I. Int. Symp. on Cyclodextrins, Budapest (1981), M. Vikom, Rapid and Simple Spectrophotometric Method for Determination of Micro-Amounts of Cyclodextrins
- Gene 47, 269-277 (1986), Binder et al.

## Beschreibung

Cyclodextrine sind Abbauprodukte der Stärke. Sie entstehen bei der Hydrolyse von Stärke durch das Enzym Cyclodextrin-Glycosyl-Transferase (CGT; E.C.2.4.1.19). Cyclodextrin-Glycosyl-Transferasen sind bisher für Bacillus macerans (z. B. ATCC 8514), Bacillus megaterium (Carbohydr. Res., 61 (1978) 229 - 238), alkalophile Bacillus-Species (z. B. ATCC 21594, 21595, 21783, 31006 und 31007) sowie bei Klebsiella pneumoniae (z. B. M5a1; Arch. microbiol., 111 (1977) 271 - 282) beschrieben worden.

CGT aus Klebsiella pneumoniae (z. B. M5a1) ist ein monomeres Polypeptid mit einem Molekulargewicht von etwa 69000 D. Dieses Polypeptid wird vom Bakterium als Vorläuferprotein mit einem Molekulargewicht von etwa 73000 D synthetisiert und unter Abspaltung eines Signalpeptids von 30 Aminosäureresten aus der Zelle ins Medium ausgeschleust.

Die Synthese des Enzyms wird strikt reguliert, insbesondere unterliegt sie einer Katabolitrepression und der Induktion.

Dies bedeutet, daß das Enzym nur synthetisiert wird, wenn beispielsweise Klebsiella pneumoniae M5a1 auf Stärke, Cyclodextrinen oder Maltooligosacchariden wächst. Wächst das Sakterium auf anderen Kohlenstoffquellen, so wird die Enzymsynthese unterdrückt bzw. nicht induziert. Da der Einsatz von Maltooligosacchariden und Cyclodextrinen aus Kostengründen nicht in Frage kommt, werden befriedigende Ausbeuten an CGT nur durch kontinuierliche Anzucht von Klebsiella pneumoniae M5a1 erzielt, wobei Stärke als Kohlenstoffquelle limitierend zugegeben werden muß. Da die Stärke aufgrund ihrer Eigenschaften (schlechte Löslichkeit, hohe Viskosität der Lösung) ein schwer handhabbares Substrat ist, ergeben sich bei der Fermentation erhebliche Probleme. Kritisch ist auch die Anwendung von CGT des potentiell pathogenen Bakteriums Klebsiella pneumoniae, insbesondere im Lebensmittelbereich und im pharmazeutischen Bereich.

Der Erfindung liegt daher die Aufgabe zugrunde, Lebendmaterial bereitzustellen, mit dem eine oder mehrere der folgenden Teilaufgaben gelöst werden können:
- CGT soll in möglichst großer Menge gebildet werden.
- Die Regulation der Enzymsynthese soll so verändert werden, daß sie leicht handhabbar wird, also eine Fermentation mit problemlosen Kohlenstoffquellen möglich macht.
- Es sollen nicht-pathogene Mikroorganismen, insbesondere Bakterien vorgesehen werden, die CGT produzieren, wobei diese Mikroorganismen das Enzym auch im Lebensmittelbereich und pharmazeutischen Bereich einer Nutzung zugänglich machen sollen.

Erfindungsgemäß werden dazu DNA-Fragmente gemäß Anspruch 1 vorgesehen. Den genannten DNA-Fragmenten können die Signalstrukturen für ihre Expression fehlen.

Die Erfindung betrifft insbesondere derartige DNA-Fragmente mit etwa 2068 Basenpaaren (bp), wobei diese DNA-Fragmente mit dem Restriktionsenzym MaeI aus DNA-Strukturen von Mikroorganismen herausschneidbar sind, die CGT produzieren. Beispiele für Spezies und Stämme derartiger Mikroorganismen wurden eingangs genannt. Der Fachmann kann sich geeignete Stämme aber auch aus Bodenproben isolieren.

Die Erfindung betrifft ferner
- Expressionsvektoren, die die genannten DNA-Fragmente umfassen;
- tac-Promotor-Plasmide, wie pKK223-3 und pJF118u; Lambda-p_{L}-Promotor-Plasmide, wie pPL-Lambda; und trp-Promotor-Plasmide, wie pDR720; die jeweils die genannten DNA-Fragmente umfassen;
- nicht-pathogene, technisch-kultivierbare Mikroorganismen, die die genannten Expressionsvektoren umfassen;
- nicht-pathogene, technisch-kultivierbare Bakterien als Beispiel für Mikroorganismen, die die genannten Expressionsvektoren umfassen; und
- derartige Mikroorganismen, insbesondere derartige Bakterien, die als Nahrungsmittelzusatz zugelassen sind.

Erfindungsgemäß wird schließlich ein Verfahren zur Herstellung der genannten DNA-Fragmente, der genannten Expressionsvektoren und der genannten Mikroorganismen vorgesehen, bei dem man
(a) von Mikroorganismen ausgewählt aus der Gruppe B. macerans, B. megaterium, alkalophile Bacillus species, und K. pnemoniae ausgeht, die CGT produzieren, deren Genom isoliert und statistisch zerkleinert, und
(b) die gewonnenen Genom-Fragmente in Vektoren einsetzt, die rekombinanten Vektoren in kultivierbare Mikroorganismen überführt, auf solche Klone screent, die CGT produzieren, und diese Klone isoliert,
(c) und/oder die rekombinanten Vektoren aus den CGT-produzierenden Klonen isoliert,
(d) und/oder DNA-Fragmente, die das CGT-Strukturgen umfassen, aus den rekombinanten Vektoren herausschneidet,
(e) und/oder die herausgeschnittenen DNA-Fragmente in Expressionsvektoren einsetzt
(f) und/oder die gewonnenen rekombinanten Expressionsvektoren in nicht-pathogene, technisch-kultivierbare Mikroorganismen überführt.

Erfindungsgemäß werden also rekombinante DNA-Methoden angewendet, um das für die Kodierung von CGT verantwortliche Gen in einen Vektor zu übertragen. Nach dem erfindungsgemäßen Verfahren kann eine hohe Kopienzahl des Gens sowie - durch gezielte Veränderung der Regulation der Genexpression - das Enzym in großer Konzentration erhalten werden. Demgemäß können der Kulturüberstand oder der Zellrohextrakt direkt oder in immobilisierter Form für die Bildung von Cyclodextrinen einsetzbar sein.

Bei der Stufe (a) geht man also von Mikroorganismen-Spezies oder -Stämmen ausgewählt aus der Gruppe B. macerans, B. megaterium, alkalophile Bacillus species, und K. pneumoniae aus, die CGT produzieren, beispielsweise von Klebsiella pneumoniae M5a1. So kann man beispielsweise bei Klebsiella pneumoniae M5a1 die Gesamt-DNA isolieren, die das Gen für CGT enthält. Dies kann durch Anzucht des Stammes in einem geeigneten Nährmedium bei 30 bis 37 ^{o}C, anschließende Lyse der Zellen und Reinigung der DNA durch Dichtegradientenzentrifugation und Dialyse geschehen.

Verwendet man also beispielsweise Klebsiella pneumoniae M5a1, so kann man deren Gesamt-DNA und auch die DNA des einzusetzenden Vektors mit der Restriktionsendonuklease PstI (E.C.3.1.23.31) bei etwa 37 ^{o}C inkubieren, bis die DNA-Moleküle vollständig gespalten sind. Anschließend kann man die Endonuklease inaktivieren. Durch Gelelektrophorese können die erhaltenen Fragmente der Gesamt-DNA aus Klebsiella pneumoniae M5a1 aufgetrennt und Fragmente mit einer Länge von etwa 5400 Basenpaaren isoliert werden.

Die Rekombination der erhaltenen etwa 5400 Basenpaare langen DNA-Bruchstücke mit dem gespaltenen Vektor kann dadurch erfolgen, daß man die Fragmente mischt und die Lösung mit einer geeigneten DNA-Ligase inkubiert, bevorzugt mit der DNA-Ligase des Phagen T4 (E.C.6.5.1.1). Die Ligation erfolgt bevorzugt in Gegenwart von ATP, einer Sulfhydryl-Verbindung und Magnesiumionen. Mit der gewonnenen rekombinanten DNA werden in bekannter Weise sogenannte kompetente Stämme transformiert, die die Fähigkeit zur Aufnahme von DNA besitzen.

Als transformierbare Stämme können im Rahmen der Erfindung die hierfür bekannten Stämme benutzt werden. Es handelt sich insbesondere um Stämme von E. coli, Klebsiella pneumoniae, Bacillus subtilis und Saccharomyces cerevisiae. Bei diesem ersten Transformationsschritt werden Stämme von E. coli, wie HB101 (DSM 1452; J. Mol. Biol., 41 (1966) 459 - 472).

Der Fachmann ist mit der Wahl von Vektoren vertraut, die für die eingesetzten, zu transformierenden Zellen geeignet sind. Bei der Verwendung von Stämmen von E. coli oder Klebsiella pneumoniae kommen für den ersten Rekombinationsschritt (b) des erfindungsgemäßen Verfahrens beispielsweise die Plasmide pBR322 (Nucl. Acids Res., 5 (1978) 2721 - 2728) und pHE3 (Gene, 19 (1982) 231 - 234) in Betracht.

Um nach dem ersten Transformationsschritt des erfindungsgemäßen Verfahrens diejenigen Zellen aufzufinden, die den das Gen für CGT tragenden Vektor enthalten, kann man die transformierten Zellen auf einem Minimalmedium anziehen, das Stärke als Kohlenstoffquelle, die notwendigen Salze und Supplemente, ein Antibiotikum zur Selektion des Vektors und Amylopectin-Azur als Indikator für Stärkeabbau enthält. Die Zellen, die den das Gen für CGT tragenden Vektor enthalten, bilden einen farblosen Abbauhof und werden isoliert.

Für einen zweiten Rekombinationsschritt des erfindungsgemäßen Verfahrens kann man bei den Stufen (c) und (d) aus den isolierten Zellen nach bekannten Methoden die Plasmid-DNA gewinnen und mit einer Restriktionsendonuklease DNA-Fragmente herausschneiden, die das CGT-Strukturgen umfassen. Ein bevorzugtes Restriktionsenzym ist MaeI (E.C.3.1.23.-), mit dem man beispielsweise bei etwa 45 ^{o}C partiell spaltet. Durch beispielsweise Gelelektrophorese werden die erhaltenen Fragmente der das Gen für CGT enthaltenden Plasmid-DNA aufgetrennt. Wurde mit der Restriktionsendonuklease MaeI geschnitten, so wird ein Fragment mit einer Länge von etwa 2068 Basenpaaren isoliert, das das Strukturgen für CGT, nicht aber die Signalstrukturen für seine Expression trägt. Die 5'-überhängenden Enden des Fragments kann man durch Inkubation mit einer geeigneten DNA-Polymerase mit Desoxy-ATP und Desoxy-TTP auffüllen, so daß glatte Enden entstehen. Bevorzugt verwendet man das Klenow-Fragment der DNA-Polymerase I (E.C.2.7.7.7).

Gemäß einer anderen Ausführungsform des beanspruchten Verfahrens kann man aber auch bei der Stufe (d) den im ersten Transformationsschritt erhaltenen Vektor mit Einer geeigneten Restriktionsendonuklease linearisieren und anschließend mit einer Exonuklease, beispielsweise mit Bal31 so lange inkubieren, bis durch schrittweise Abspaltung von Nukleotiden von den Enden her ein Fragment entsteht, das das Gen für CGT enthält, vorzugsweise ohne seine Signalstrukturen. Überhängende Enden des Fragments kann man mit einer DNA-Polymerase mit Desoxynukleotiden auffüllen, so daß glatte Enden entstehen.

Für den zweiten Rekombinationsschritt des erfindungsgemäßen Verfahrens, nämlich die Stufe (e), kann man sogenannte Expressionsvektoren verwenden, die gut steuerbare regulatorische Sequenzen enthalten und eine hohe Expression des rekombinierten Gens bewirken. Für die Transformation von Stämmen von E. coli und Klebsiella pneumoniae können beispielsweise tac-Promotorplasmide verwendet werden, wie pKK223-3 (im Handel erhältlich) und pJF118u; pJF118u entspricht pKK223-3 mit den Ausnahmen, daß ein 0.546 kb BamHI-XmaIII-Fragment deletiert und ein 1.26 kb-Fragment mit Lactose-Repressor-Gen (lacI^{q}) inseriert wurde. Es können jedoch auch andere Plasmide, beispielsweise Lambda-p_{L}-Promotor-Plasmide, wie pPL-Lambda (im Handel erhältlich), und trp-Promotor-Plasmide verwendet werden, wie pDR720 (im Handel erhältlich).

Die Spaltung der Vektor-DNA erfolgt mit einer geeigneten Restriktionsendonuklease. Anschließend kann man die Restriktionsendonuklease inaktivieren. Überhängende Enden kann man mit Hilfe einer geeigneten DNA-Polymerase mit Desoxynukleotiden auffüllen oder abspalten, so daß glatte Enden entstehen. Bevorzugt kann man dafür das Klenow-Fragment der DNA-Polymerase I oder die DNA-Polymerase des Phagen T4 verwenden.

Die Rekombination des DNA-Fragments, das das CGT-Strukturgen umfaßt, mit der Vektor-DNA kann man dadurch erreichen, daß man die Fragmente mischt und die Lösung mit einer geeigneten DNA-Ligase inkubiert, bevorzugt mit DNA-Ligase des Phagen T4 (aus E.C.6.5.1.1). Die Ligation erfolgt zweckmäßigerweise in Gegenwart von ATP, einer Sulfhydryl-Verbindung und Magnesiumionen.

Gemäß Stufe (f) des erfindungsgemäßen Verfahrens kann man die erhaltene rekombinante DNA in bekannter Weise in sogenannte kompetente Stämme transformieren, die die Fähigkeit zur Aufnahme von DNA besitzen. Bei diesem zweiten Transformationsschritt des erfindungsgemäßen Verfahrens erfolgt die Auswahl der transformierbaren Zellen nach gewünschten sonstigen Eigenschaften dieser Zellen, wie beispielsweise Eigenschaften des Wachstums, der Pathogenität und der Fähigkeit zum Export von Proteinen. Bevorzugt werden Stämme von E. coli, wie HB101 und JM 105 (im Handel erhältlich), und Klebsiella pneumoniae, wie UN1290 (J. Bacteriol., 136 (1978) 253 - 266). Es können jedoch auch transformierbare Zellen anderer Stämme verwendet werden, wie beispielsweise von Saccharomyces cerevisiae und Bacillus subtilis.

Nach der Transformation kann man diejenigen Zellen, die Plasmide enthalten, auf einem Nährmedium mit einem Gehalt an einem Antibiotikum anziehen. Und zwar kann man diejenigen Zellen, die ein Plasmid enthalten, parallel einerseits auf einem Minimalmedium, welches Glukose als Kohlenstoffquelle, die notwendigen Salze und Supplemente, ein Antibiotikum zur Selektion des Vektors und Amylopektin-Azur als Indikator für Stärkeabbau enthält, und andererseits auf dem gleichen Minimalmedium anziehen, das zusätzlich noch einen Induktor enthält, beispielsweise Isopropyl-β-D-thiogalactosid im Fall des Vektors pKK223-3. Diejenigen Zellen, die mit dem Induktor einen farblosen Abbauhof bilden und damit offensichtlich unter der Kontrolle des Promotors stehen, werden isoliert. Sie besitzen das Strukturgen für CGT, das unter der Kontrolle des Expressionspromotors steht. Im Fall des Vektors pKK223-3 handelt es sich um den tac-Promotor.

Bei den Stufen (d) und (e) des erfindungsgemäßen Verfahrens ist es für den zweiten Rekombinationsschritt vorteilhaft, die partielle Spaltung der nach der ersten Transformation erhaltenen Plasmid-DNA mit der Restriktionsendonuklease MaeI durchzuführen. Dieses Restriktionsenzym bewirkt nämlich eine Spaltung an vorteilhaften Stellen der DNA, und zwar einerseits zwischen der Ribosomen-Bindungsstelle und der Startstelle der Translation und andererseits hinter den Terminationssignalen für Transskription und Translation der das Gen für CGT enthaltenden DNA-Sequenz.

Es sei nochmals betont, daß die Erfindung neben den DNA-Fragmenten, die das CGT-Strukturgen umfassen und die nach dem erfindungsgemäßen Verfahren gewonnen werden können, auch DNA-Fragmente umfaßt, mit deren Hilfe Enzyme mit CGT-Leistung exprimiert werden können und deren Einzelstränge mit denen der DNA-Fragmente mit dem CGT-Strukturgen von Microorganismen ausgewählt aus der Gruppe B. macerans, B. megaterium, alkalophile Bacillus species, und K. pneumoniae bei mindestens 20°C und insbesondere bei einer Konzentration von 1 M NaCl und einer Temperatur von mindestens 25°C hybridisierbar sind. Derartige "hybridisierbare" DNA-Fragmente können beispielsweise durch Modifikation der DNA-Fragmente anfallen, die das CGT-Strukturgen umfassen. Mit der Ergänzung der Erfindung um diese "hybridisierbaren" DNA-Fragmente wird auch dem Umstand Rechnung getragen, daß sich die Strukturgene der Mikroorganismen unterscheiden können, die CGT produzieren. Modifizierte DNA-Fragmente sind insofern von Interesse, als mit ihrer Hilfe Enzyme mit CGT-Leistung exprimiert werden können.

Der Mikroorganismus Klebsiella pneumoniae M5a1 ist unter der Nummer DSM 3539 bei der Deutschen Sammlung von Mikroorganismen in D-3400 Göttingen, Grisebachstraße 8, hinterlegt.

Nachstehend wird die Erfindung durch eine Schemazeichnung und ein Beispiel näher erläutert.

### Beispiel 1

Aus der Gesamt-DNA des das Gen für Cyclodextrin-Glycosyl-Transferase tragenden Stammes Klebsiella pneumoniae M5a1 werden neue Stämme mit hoher Produktivität für Cyclodextrin-Glycosyl-Transferase durch folgende Verfahrensschritte hergestellt:
(1) Isolierung von Gesamt-DNA aus Klebsiella pneumoniae M5a1, die die genetische Information für die Cyclodextrin-Glycosyl-Transferase enthält. Der Stamm Klebsiella pneumoniae M5a1 wird in 200 ml Nährbouillon, die 1 % Trypton, 0.5 % Hefeextrakt und 1 % NaCl enthält, bei 37°C bis zum Ende des exponentiellen Phase angezogen. Nach der Ernte werden die Zellen in 2.5 ml TES-Puffer (50 mM Tris/Cl pH 7.5; 50 mM NaCl; 5 mM EDTA), der 25 % Saccharose und 5 mg Lysozym enthält, aufgenommen und 10 Minuten lang bei 37°C inkubiert. Nach Zugabe von 2.5 ml Lysislösung (10 mM Tris/Cl pH 7.5; 1 mM EDTA; 0.1 % Triton X100) wird der Ansatz mit Proteinase K zu einer Endkonzentration von 0.1 mg/ml versetzt und 30 Minuten lang bei 37°C inkubiert. Die DNA wird aus dem Lysat durch Cäsiumchlorid-Dichtegradientenzentrifugation und Dialyse gegen Puffer (10 mM Tris/Cl pH 8.0; 1 mM EDTA) isoliert.
(2) Isolierung der Vektor DNA.
   Zur Klonierung des Gens für die Cyclodextrin-Glycosyl-Transferase wird DNA des Plasmids pBR322, das Ampicillin- und Tetracyclin-Gene als Marker trägt, verwendet. Es wird in folgender Weise isoliert:
   Ein E. coli Stamm, der das Plasmid pBR322 enthält, wird bei 37°C in 0.5 ml Nährbouillon wie unter (1) bis zum Ende der exponentiellen Wachstumsphase unter Schütteln angezogen. Nach Zugabe von 150 »g/ml Chloramphenicol wird 15 Stunden lang bei der gleichen Temperatur weitergeschüttelt. Anschließend werden die Bakterienzellen gewonnen, mit Lysozym und einem nichtionischen Detergens lysiert und das Lysat bei 48 000 g 30 Minuten lang zentrifugiert. Aus dem Überstand wird die Plasmid-DNA mit Hilfe zweimaliger Cäsiumchlorid-Gleichgewichtsdichtegradientenzentrifugation und Dialyse gegen Puffer wie unter (1) gewonnen.
(3) Insertion des für Cyclodextrin-Glycosyl-Transferase kodierenden Gens aus Klebsiella pneumoniae M5a1 in den Vektor.
   10 »g der Gesamt-DNA aus Klebsiella pneumoniae M5a1 und 1 »g der Vektor DNA werden zwei Stunden lang mit der Restriktionsendonuklease PstI behandelt, um die DNA-Moleküle komplett zu spalten, und dann jeweils 10 Minuten auf 68°C erhitzt. Die Gesamt-DNA aus Klebsiella pneumoniae wird danach in einem 0.6%igen Agarosegel durch Elektrophorese aufgetrennt. Die Fragmente mit einer Größe von etwa 5 400 Basenpaaren werden ausgeschnitten und nach Phenolextraktion und Ethanolfällung in Wasser gelöst.
   Die Lösung der so gewonnenen Fragmente der Gesamt-DNA von Klebsiella pneumoniae M5a1 wird mit der Lösung der gespaltenen pBR322 Vektor DNA vermischt und nach Zugabe von ATP, Dithiothreitol und Magnesiumchlorid 16 Stunden lang mit der DNA-Ligase des Phagen T4 bei 17°C inkubiert. Die erhaltene Lösung enthält die rekombinante DNA.
(4) Genetische Transformation von E. coli-Bakterien mit rekombinanter DNA, die die genetische Information für Cyclodextrin-Glycosyl-Transferase enthält.
   Der E. coli Stamm HB101 wird unter Schütteln in 20 ml Nährbouillon wie unter (1) bei 37°C bis zum Ende der exponentiellen Phase angezogen. Die Zellen werden gewonnen, in 10 ml eiskalter 50 mM Calciumchlorid-Lösung suspendiert und 30 Minuten im Eisbad inkubiert. Nach anschließender Gewinnung durch Zentrifugation werden die Zellen in 2 ml eiskalter 50 mM Calciumchlorid-Lösung suspendiert. 0.2 ml der Suspension werden mit der Lösung der rekombinanten DNA aus Stufe (3) gemischt, 30 Minuten lang im Eisbad und anschließend 5 Minuten lang bei 43°C inkubiert. Die Zellen werden in Nährbouillon wie unter (1) beimpft und 45 Minuten lang bei 37°C geschüttelt. Anschließend werden die Zellen in Portionen auf Agarplatten ausgestrichen, die pro Liter 7.1 g Na₂HPO₄, 13.6 g KH₂PO₄, 15 mg CaCl₂, 0.25 g MgSO₄, 1.6 g (NH₄)₂SO₄, 5 mg Thiamin, 40 mg Prolin, 40 mg Leucin, 20 mg Tetracyclin, 5 g Stärke, 5 g Amylopectin-Azur und 15 g Agar enthalten. Die Platten werden bei 37°C bebrütet. Nach drei Tagen Bebrütung erscheinen Kolonien auf den Platten, von denen einige von farblosen Höfen auf den ansonsten tiefblauen Platten umgeben sind. Die letztgenannten Kolonien werden entnommen, gereinigt und isoliert. Jede der so gewonnenen Kolonien ist zum Abbau von Stärke befähigt und gleichzeitig Tetracyclin-resistent. Sie hat damit andere Eigenschaften als der Stamm, der als Wirtsorganismus verwendet wird. Das bedeutet, daß die isolierten Zellen das Plasmid pBR322 enthalten, das das PstI-Fragment aus Klebsiella pneumoniae M5a1 mit dem Cyclodextrin-Glycosyl-Transferase-Gen trägt.
   Aus einer der erhaltenen Kolonien wird die Plasmid-DNA nach dem unter (2) beschriebenen Verfahren isoliert. Nach Behandlung mit Restriktionsendonuklease PstI und Analyse durch Elektrophorese in einen Agarosegel zeigen sich zwei Fragmente. Dieses Plasmid wird pCM100 benannt.
   Bakterien, die dieses Plasmid tragen, können Stärke abbauen und produzieren Cyclodextrin-Glycosyl-Transferase.
(5) 10 »g der DNA des Plasmids pCM100 aus HB101 werden mit der Restriktionsendonuklease MaeI bei 45°C so behandelt, daß die DNA-Moleküle partiell gespalten werden. Durch Elektrophorese in einem 1%igem Agarosegel werden die DNA-Fragmente aufgetrennt. Das Fragment mit einer Länge von 2 068 Basenpaaren wird ausgeschnitten und wie unter (3) isoliert.
   Die 5'-überhängenden Enden werden durch Inkubation mit dem sogenannten Klenow-Fragment der DNA-Polymerase I mit Desoxy-ATP und Desoxy-TTP bei 25°C aufgefüllt, so daß glatte Enden entstehen.
   Die Restriktionsendonuklease MaeI spaltet die DNA des Plasmids pCM100 einerseits zwischen der Ribosomenbindungsstelle und der Startstelle der Translation und andererseits hinter den Terminationssignalen für Transkription und Translation der das Gen für die Cyclodextrin-Glycosyl-Transferase enthaltenden DNA-Sequenz. Eine partielle Spaltung der DNA des Plasmids pCM100 ist notwendig, da die Restriktionsendonuklease innerhalb des Gens für Cyclodextrin-Glycosyl-Transferase eine weitere Schnittstelle erkennt.
(6) Isolierung der DNA eines Expressionsvektors.
   Zur Klonierung des Strukturgens der Cyclodextrin-Glycosyl-Transferase in einen Expressionsvektor wird DNA des Plasmids pJF118u, das das Ampicillin-Gen als Marker, einen tac-Promotor und das Gen für einen lac-Repressor trägt, eingesetzt. Die Plasmid-DNA wird nach der unter (2) beschriebenen Methode isoliert.
(7) Insertion des für Cyclodextrin-Glycosyl-Transferase kodierenden Strukturgens aus Klebsiella pneumoniae M5a1 in den Expressionsvektor (siehe Schemazeichnung).
   1 »g der DNA des Plasmids pJF118u wird mit Restriktionsendonuklease EcoRI vollständig bei 37° C gespalten. Die Endonuklease wird anschließend inaktiviert und die 5'-überhängenden Enden werden durch Inkubation mit dem sogenannten Klenow-Fragment der DNA-Polymerase I mit Desoxy-ATP und Desoxy-TTP bei 25°C aufgefüllt, so daß glatte Enden entstehen. Die Lösung des unter (5) erhaltenen MaeI-Fragments mit einer Länge von 2 068 Basenpaaren wird mit der gespaltenen pJF118u-Vektor-DNA gemischt und nach Zugabe von ATP, Dithiothreitol und Magnesiumchlorid 16 Stunden lang bei 17°C mit der DNA-Ligase des Phagen T4 inkubiert. Die erhaltene Lösung enthält die rekombinante DNA.
(8) Genetische Transformation von E. coli Bakterien mit rekombinanter DNA, die die genetische Information für Cyclodextrin-Glycosyl-Transferase unter Kontrolle des tac-Promotors enthält.

Der E. coli Stamm HB101 wird wie unter (4) beschrieben angezogen und zur Transformation vorbereitet. 0.2 ml der wie unter (4) erhaltenen Suspension werden mit der Lösung der rekombinanten DNA aus Stufe (7), wie unter (4) beschrieben, transformiert. Anschließend werden die Zellen auf Agarplatten ausgestrichen, die pro Liter 10 g Trypton, 5 g Hefeextrakt, 10 g NaCl, 100 mg Ampicillin und 15 g Agar enthalten. Die Platten werden bei 37° C bebrütet. Nach 24 Stunden Bebrütung erscheinen auf den Platten Kolonien, die gegen Ampicillin resistent sind. Sie werden abgenommen und parallel auf Agarplatten ausgestrichen, die einerseits pro Liter 7.1 g Na₂HPO₄, 13.6 g KH₂PO₄, 15 mg CaCl₂, 0.25 g MgSO₄, 1.6 g (NH₄)₂SO₄, 5 mg Thiamin, 40 mg Prolin, 40 mg Leucin, 100 mg Ampicillin, 5 g Glukose, 5 g Amylopectin-Azur und 15 g Stärke, andererseits die gleichen Bestandteile, aber zusätzlich noch 238 mg Isopropyl-β-D-Thiogalactosid als Induktor enthalten. Die Platten werden bei 37° C bebrütet. Nach 24 Stunden Bebrütung erscheinen Kolonien auf den Platten, von denen einige auf den Platten, die den Induktor enthalten, von farblosen Höfen auf den sonst tiefblauen Platten umgeben sind. Letztgenannte Kolonien werden abgenommen, gereinigt und isoliert.

Jede der so gewonnenen Kolonien ist nach Induktion mit Isopropyl-β-D-Thiogalactosid zum Stärkeabbau befähigt und außerdem Ampicillin-resistent.

Das bedeutet, daß die isolierten Zellen das Plasmid pJF118u enthalten, das das MaeI-Fragment aus pCM100 mit dem Gen für Cyclodextrin-Glycosyl-Transferase trägt, welches unter Kontrolle des tac-Promotors steht.

Das Plasmid wird pCM301 benannt.

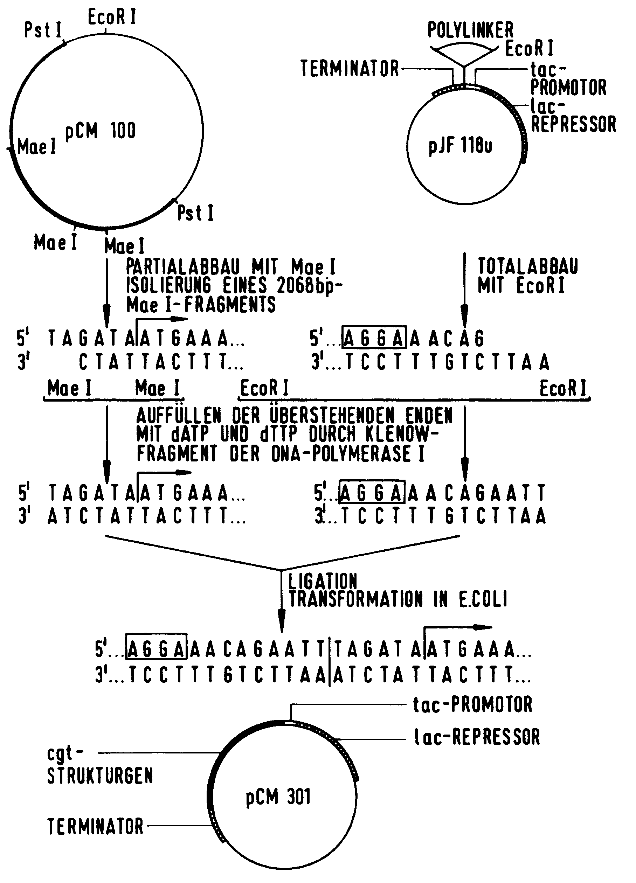

## Patentansprüche (Patentansprüche für folgende(n) Vertragsstaat(en): CH, DE, FR, GB, IT, LI, NL, SE)

1. DNA-Fragment, umfassend ein Cyclodextrin-Glycosyl-Transferase-Strukturgen (CGT-Strukturgen) mit einer Größe von etwa 2100 bp, herausschneidbar mittels partiellen Abbaus mit dem Restriktionsenzym Mae I und anschließendem Exonuclease Verdau aus DNA-Strukturen von Stämmen der folgenden Spezies, die CGT produzieren: B. macerans, Bacillus megaterium, alkalophile Bacillus-Spezies oder Klebsiella pneumoniae und DNA-Fragmente, mit deren Hilfe Enzyme mit CGT-Leistung exprimiert werden können und deren Einzelstränge mit denen obiger CGT-Strukturgene bei mindestens 20°C und insbesondere bei einer Konzentration von 1 M NaCl und einer Temperatur von mindestens 25°C hybridisierbar sind.

2. DNA-Fragment nach Anspruch 1 einschließlich des das Leaderpeptid (Signalpeptid) kodierenden DNA-Bereichs, wobei dem DNA-Fragment Signalstrukturen für seine Expression fehlen.

3. Expressionsvektor, umfassend ein DNA-Fragment gemäß einem der Ansprüche 1 bis 2.

4. tac-Promotor-Plasmide wie
pKK223-3 und
das aus pKK223-3 durch Deletion eines 546 bp BamHI-XmaIII-Fragments sowie Insertion eines 1.26 kb Fragments enthaltend das Laktose-Repressor-Gen erhältliche Plasmid Lambda-p_{L}-Promotor-Plasmide wie
pPL-Lambda; und
trp-Promotor-Plasmide wie
pDR720;
jeweils umfassend ein DNA-Fragment gemäß einem der Ansprüche 1 bis 2.

5. Nicht-pathogene, technisch-kultivierbare Mikroorganismen, umfassend einen Expressionsvektor gemäß Anspruch 3 oder 4.

6. Nicht-pathogene, technisch-kultivierbare Bakterien, umfassend einen Expressionsvektor gemäß Anspruch 3 oder 4.

7. Mikroorganismen nach Anspruch 5 oder 6 und zugelassen als Nahrungsmittelzusatz.

8. Verfahren zur Herstellung von
- DNA-Fragmenten gemäß einem der Ansprüche 1 bis 2,
- Expressionsvektoren gemäß Anspruch 3 oder 4 und
- Mikroorganismen gemäß einem der Ansprüche 5 bis 7,
dadurch gekennzeichnet, daß man
(a) von Mikroorganismen ausgewählt aus den Stämmen der folgenden Spezies Bacillus macerans, Bacillus megaterium alkalophile Bacillus species oder Klebsiella pneumoniae, ausgeht, die CGT produzieren, deren Genom isoliert und statistisch zerkleinert,
(b) die gewonnenen Genom-Fragmente in Vektoren einsetzt, die Vektoren in kultivierbare Mikroorganismen überführt, auf solche Klone screent, die CGT produzieren, und diese Klone isoliert,
(c) und/oder die Vektoren aus den CGT-produzierenden Klonen isoliert,
(d) und/oder DNA-Fragmente gemäß einem der Ansprüche 1 bis 2 aus den Vektoren herausschneidet
(e) und/oder die herausgeschnittenen DNA-Fragmente in Expressionsvektoren einsetzt
(f) und/oder die gewonnenen Expressionsvektoren in nicht-pathogene, technisch-kultivierbare Mikroorganismen überführt.

9. Verfahren nach Anspruch 8, dadurch gekennzeichnet, daß man bei der Stufe (e) DNA-Fragmente verwendet, deren Einzelstränge mit den Einzelsträngen der bei der Stufe (d) herausgeschnittenen DNA-Fragmente bei mindestens 20° C und insbesondere bei einer Konzentration von 1 M NaCl und einer Temperatur von mindestens 25° C hybridisierbar sind.

## Patentansprüche (Patentansprüche für folgende(n) Vertragsstaat(en): AT)

1. Verfahren zur Herstellung von
- einem DNA-Fragment, umfassend das Cyclodextrin- Glycosyl-Transferase-Strukturgen (CGT-Strukturgen) mit einer Größe von etwa 2100 bp herausschneidbar aus DNA-Strukturen mittels partiellen Abbaus mit dem Restriktionsenzym Mae I und anschließendem Exonuklease Verdau und DNA-Fragmenten, mit deren Hilfe Enzyme mit CGT-Leistung exprimiert werden können und deren Einzelstränge mit denen des DNA-Fragments, das das CGT-Strukturgen umfaßt bei mindestens 20°C und insbesondere bei einer Konzentration von 1 M NaCl und einer Temperatur von mindestens 25°C, hybridisierbar sind,
- Expressionsvektoren, die die genannten DNA-Fragmente umfassen,
-tac-Promotor-Plasmiden, wie pKK 223-3 und dem aus pkk223-3 durch Deletion eines 546 bp BamHI-XmaIII-Fragments sowie Insertion eines 1.26 kb Fragments enthaltend das Laktose-Repressor-Gen erhältlichen Plasmid, Lambda-p_{L}-Promotor-Plasmiden, wie pPL-Lambda und trp-Promotor-Plasmiden, wie pDR720, die jeweils die genannten Expressionsvektoren umfassen,
- nicht pathogenen, technisch-kultivierbaren Mikroorganismen, die die genannten Expressionsvektoren umfassen,
- nicht pathogenen, technisch-kultivierbaren Bakterien als Beispiele für Mikroorganismen, die die genannten Expressionsvektoren umfassen und
- derartigen Mikroorganismen, insbesondere derartigen Bakterien, die als Nahrungsmittelzusatz zugelassen sind, dadurch gekennzeichnet, daß man
(a) von Mikroorganismen ausgewählt aus den Stämmen der folgenden Spezies Bacillus macerans, Bacillus megaterium, alkalophile Bacillus-Spezies oder Klebsiella pneumoniae ausgeht, die CGT produzieren, deren Genom isoliert und statistisch zerkleinert,
(b) die gewonnenen Genom-Fragmente in Vektoren einsetzt, die Vektoren in kultivierbare Mikroorganismen überführt, auf solche Klone screent, die CGT produzieren und diese Klone isoliert, (c) und/oder die Vektoren aus den CGT-produzierenden Klonen isoliert,
(d) und/oder die herausgeschnittenen DNA-Fragmente in Expressionsvektoren einsetzt
(f) und/oder die gewonnenen Expressionsvektoren in nicht-pathogene, technisch-kultivierbare Mikroorganismen überführt.

2. Verfahren nach Anspruch 1, **dadurch gekennzeichnet,** daß man bei der Stufe (d) mit dem Restriktionsenzym MaeI schneidet.

3. Verfahren nach Anspruch 1 oder 2, **dadurch gekennzeichnet,** daß man bei der Stufe (d) die Vektoren mit einer Restriktionsendonuklease linearisiert und mit einer Exonuklease zu einem DNA-Fragment abbaut.

4. Verfahren nach Anspruch 3, **dadurch gekennzeichnet,** daß man Bal31 als Exonuklease verwendet.

5. Verfahren nach einem der Ansprüche 1 bis 4, dadurch gekennzeichnet, daß man bei der Stufe (e) tac-Promotor Plasmide (wie pKK223-3 und das aus pKK223-3 durch Deletion eines 546 bp BamHI-XmaIII-Fragments sowie Insertion eines 1.26 kb Fragments enthaltend das Laktose-Repressor-Gen erhältliche Plasmid), Lambda-p_{L}-Promotor-Plasmide (wie pPL-Lambda) und trp-Promotor-Plasmide (wie pDR720) verwendet.

6. Verfahren nach einem der Ansprüche 1 bis 5, **dadurch gekennzeichnet,** daß man bei der Stufe (e) DNA-Fragmente verwendet, deren Einzelstränge mit den Einzelsträngen der bei der Stufe (d) herausgeschnittenen DNA-Fragmente bei mindestens 20° C und insbesondere bei einer Konzentration von 1 M NaCl und einer Temperatur von mindestens 25° C hybridisierbar sind.

## Claims (Claims for the following Contracting State(s): CH, DE, FR, GB, IT, LI, NL, SE)

1. DNA fragment embracing a cyclodextrin glycosyl-transferase structural gene (CGT structural gene) with a size of about 2,100 bp, which can be cut out by means of partial degradation with the restriction enzyme MaeI and subsequent exonuclease digestion from DNA structures of strains of the following species which produce CGT: B. macerans, Bacillus megaterium, alkalophilic Bacillus species or Klebsiella pneumoniae and DNA fragments with the aid of which enzymes with CGT power can be expressed and whose single strands are able to hybridise with those of the above CGT structural genes at not below 20°C and in particular at a concentration of 1M NaCl and a temperature not below 25°C.

2. DNA fragment according to Claim 1, including the DNA region coding for the leader peptide (signal peptide), there being missing from the DNA fragment the signal structures for its expression.

3. Expression vector embracing a DNA fragment according to one of Claims 1 to 2.

4. tac promoter plasmids such as pKK223-3 and the plasmid obtainable from pkk223-3 by deletion of a 546 bp BamHI-XmaIII fragment and insertion of a 1.26 kb fragment containing the lactose repressor gene;
lambda-p_{L} promoter plasmids such as
pPL-lambda; and
trp promoter plasmids such as
pDR720;
each embracing a DNA fragment according to one of Claims 1 to 2.

5. Non-pathogenic micro-organisms which embrace an expression vector according to Claim 3 or 4 and which can be cultivated industrially.

6. Non-pathogenic bacteria which embrace an expression vector according to Claim 3 or 4 and which can be cultivated industrially.

7. Micro-organisms according to Claim 5 or 6 and approved for addition to foodstuffs.

8. Process for the preparation of
- DNA fragments according to one of Claims 1 to 2,
- expression vectors according to Claim 3 or 4 and
- micro-organisms according to one of Claims 5 to 7,
characterised in that
(a) starting from micro-organisms which are selected from the strains of the following species Bacillus macerans, Bacillus megaterium alkalophilic Bacillus species or Klebsiella pneumoniae, and which produce CGT, their genome is isolated and randomly fragmented, and
(b) the genome fragments which have been obtained are inserted in vectors, the vectors are transferred into micro-organisms which can be cultivated, screening is carried out for those clones which produce CGT, and these clones are isolated,
(c) and/or the vectors are isolated from the CGT-producing clones,
(d) and/or DNA fragments according to one of Claims 1 to 2 are cut out of the vectors,
(e) and/or the DNA fragments which have been cut out are inserted in expression vectors,
(f) and/or the expression vectors which have been obtained are transferred into non-pathogenic micro-organisms which can be cultivated industrially.

9. Process according to Claim 8, characterised in that there is use, in stage (e), of DNA fragments whose single strands are able, at not below 20°C and in particular at a concentration of 1 M NaCl and a temperature not below 25°C, to hybridise with the single strands of the DNA fragments cut out in stage (d).

## Claims (Claims for the following Contracting State(s): AT)

1. Process for the preparation of
- a DNA fragment embracing the cyclodextrin glycosyltransferase structural gene (CGT structural gene) with a size of about 2,100 bp, which can be cut out from DNA structures by means of partial degradation with the restriction enzyme Mae I and subsequent exonuclease digestion, and DNA fragments with the aid of which enzymes with CGT power can be expressed and whose single strands are able to hybridise with those of the DNA fragment which embraces the CGT structural gene at not below 20°C and in particular at a concentration of 1 M NaCl and a temperature not below 25°C,
- expression vectors which embrace the said DNA fragments,
- tac promoter plasmids such as pKK 223-3 and the plasmid obtainable from pkk223-3 by deletion of a 546 bp BamHI-XmaIII fragment and insertion of a 1.26 Kb fragment containing the lactose repressor gene, lambda-p_{L} promoter plasmids such as pPL-lambda and trp promoter plasmids such as pDR720, which each embrace the said expression vectors,
- non-pathogenic micro-organisms which embrace the said expression vectors and can be cultivated industrially,
- non-pathogenic bacteria as examples of micro-organisms which embrace the said expression vectors and which can be cultivated industrially and
- micro-organisms of this type, in particular bacteria of this type, which are approved for addition to foodstuffs, characterised in that
(a) starting from micro-organisms which are selected from the strains of the following species Bacillus macerans, Bacillus megaterium alkalophilic Bacillus species or Klebsiella pneumoniae, and which produce CGT, their genome is isolated and randomly fragmented, and
(b) the genome fragments which have been obtained are inserted in vectors, the vectors are transferred into micro-organisms which can be cultivated, screening is carried out for those clones which produce CGT, and these clones are isolated, (c) and/or the vectors are isolated from the CGT-producing clones,
(d) and/or the DNA fragments which have been cut out are inserted in expression vectors,
(f) and/or the expression vectors which have been obtained are transferred into non-pathogenic micro-organisms which can be cultivated industrially.

2. Process according to Claim 1, characterised in that cutting is carried out in stage (d) with the restriction enzyme MaeI.

3. Process according to Claim 1 or 2, characterised in that in stage (d) the vectors are linearised with a restriction endonuclease and degraded with an exonuclease to give a DNA fragment.

4. Process according to Claim 3, characterised in that Bal31 is used as exonuclease.

5. Process according to one of Claims 1 to 4, characterised in that in stage (e) tac promoter plasmids (such as pKK223-3 and the plasmid obtainable from pKK223-3 by deletion of a 546 bp BamHI-XmaIII fragment and insertion of a 1.26 kb fragment containing the lactose repressor gene), lambda-p_{L} promoter plasmids (such as pPL-lambda) and trp promoter plasmids (such as pDR720) are used.

6. Process according to one of Claims 1 to 5, characterised in that there is use, in stage (e), of DNA fragments whose single strands are able, at not below 20°C and in particular at a concentration of 1 M NaCl and a temperature not below 25°C, to hybridise with the single strands of the DNA fragments cut out in stage (d).

## Revendications (Revendications pour l'(les) Etat(s) contractant(s) suivant(s): CH, DE, FR, GB, IT, LI, NL, SE)

1. Fragment d'ADN, comprenant un gène de structure de cyclodextrine-glycosyl-transférase (gène de structure de CGT) ayant une grandeur d'environ 2100 paires de bases, que l'on peut découper au moyen d'une dégradation partielle par l'enzyme de restriction MaeI puis digestion par de l'exonucléase à partir de structures d'ADN de souche des espèces suivantes, qui produisent la CGT : Bacillus macerans, Bacillus megatérium, les espèces alcalophiles de Bacillus ou Klebsiella pneumoniae et fragments d'ADN à l'aide desquels on peut exprimer des enzymes ayant les caractéristiques de la CGT et dont les brins individuels peuvent être hybridés avec les gènes de structure de la CGT ci-dessus à 20° C au moins et en particulier à une concentration de NaCl de 1 M et à une température d'au moins 25°C.

2. Fragment d'ADN selon la revendication 1, y compris les zones d'ADN codant pour le peptide de tête (peptide de signal), le fragment d'ADN étant dépourvu de structures de signal pour son expression.

3. Vecteur d'expression, comprenant un fragment d'ADN selon l'une des revendications 1 et 2.

4. Plasmides promoteurs tac comme pKK223-3 et plasmides que l'on peut obtenir à partir de pKK223-3 par abandon d'un fragment BamHI-XmaIII de 546 paires de bases et insertion d'un fragment de 1,26 kb contenant le gène répresseur de lactose, plasmides promoteurs de type Lambda-p_{L} comme pPL-Lambda ; et plasmides promoteurs trp comme pDF720, chacun comprenant un fragment d'ADN selon l'une des revendications 1 et 2.

5. Micro-organismes non pathogènes, cultivebles à l'échelle technique ou industrielle, comprenant un vecteur d'expression selon la revendication 3 ou 4.

6. Bactéries non pathogènes, cultivables à l'échelle technique ou industrielle, comprenant un vecteur d'expression selon la revendication 3 ou 4.

7. Micro-organismes selon les revendications 5 ou 6 et autorisés comme additifs pour aliments.

8. Procédé pour préparer :
- des fragments d'ADN selon l'une des revendications 1 et 2,
- des vecteurs d'expression selon la revendication 3 ou 4, et
- des micro-organismes selon l'une des revendications 5 à 7, caractérisés en ce que :
(a) on part de micro-organismes choisis parmi les souches des espèces suivantes : Bacillus macerans, Bacillus megaterium, une espèce de Bacillus alcalophile ou Klebsiella pneumoniae, qui produisent de la CGT, on en isole le génome et on le fragmente statistiquement,
(b) on introduit les fragments de génome ainsi obtenus dans des vecteurs, on transforme les vecteurs en des micro-organismes cultivables, on recherche les clones produisant de la CGT et l'on isole ces clones,
(c) et/ou on isole les vecteurs à partir des clones producteurs de la CGT,
(d) et/ou on découpe sur les vecteurs des fragments d'ADN selon l'une des revendications 1 et 2,
(e) et/ou on introduit les fragments d'ADN, ainsi découpés dans des vecteurs d'expression,
(f) et/ou on transforme les vecteurs d'expression ainsi obtenus en des micro-organismes non pathogènes, cultivables à l'échelle technique ou industrielle.

9. Procédé selon la revendication 8, caractérisé en ce que, dans l'étape (e) on utilise des fragments d'ADN dont les brins individuels sont hybridables avec les brins individuels des fragments d'ADN découpés à l'étape (d), à 20°C au moins et en particulier à une concentration de NaCl de 1 M et à une température d'au moins 25°C.

## Revendications (Revendications pour l'(les) Etat(s) contractant(s) suivant(s): AT)

1. Procédé pour la préparation
- d'un fragment d'ADN englobant le gène de structure de la cyclodextrine-glycosyle-transférase (gène de structure CGT) ayant une taille d'environ 2100 pb que l'on peut découper des structure d'ADN par dégradation partielle avec une enzyme de restriction Mae I et ensuite par digestion avec une exonucléase, et de fragments d'ADN à l'aide desquels on peut exprimer des enzymes aynt des caractéristiques de la CGT et dont les brins individuels avec ceux du fragment d'ADN qui englobe le gène de structure CGT peuvent être hybridés à au moins 20°C et notamment à une concentration de NaCl de 1M et une température d'au moins 25°C,
- de vecteurs d'expression qui englobent les fragments d'ADN cités,
- des plasmides du promoteur tac, comme le plasmide pKK 223-3 et celui du pKK 223-3 que l'on peut obtenir par déletion d'un fragment BamHI-XmaIII de 546 pb ainsi que l'insertion d'un fragment de 1,26 kb contenant le gène répresseur du lactose, des plasmides du promoteur lambda-pL ainsi que des plasmides pPl-lambda et du promoteur trp, comme pDR720, qui englobent chacun les vecteurs d'expression cités,
- des micro-organismes non pathogènes, cultivables industriellement qui englobent les vecteurs d'expression cités, et
- des micro-organismes, notamment des bactéries, du type autorisé comme additifs alimentaires, caractérisé en ce que
(a) on part des micro-organismes choisis parmi les souches des espèces suivantes : Bacillus macerans, Bacillus megaterium, des espèces alcalophiles de Bacillus ou Klebsiella pneumoniae, qui produisent la CGT dont on isole et fragmente statistiquement le génome,
(b) on utilise dans des vecteurs les fragments du génome obtenus, on transfère les vecteurs dans des micro-roganismes cultivables, on crible pour déceler des clones qui produisent la CGT et on isole ces clones,
(c) et/ou on isole les vecteurs à partir des clones produisant la CGT,
(d) et/ou on utilise dans les vecteurs d'expression les fragments d'ADN découpés,
(f) et/ou on transfère les vecteurs d'expression obtenus dans des micro-organismes non pathogènes, cultivables industriellement.

2. Procédé selon la revendication 1, caractérisé en ce qu'à l'étape (d), on découpe à l'aide de l'enzyme de restriction MaeI.

3. Procédé selon la revendication 1 ou 2, caractérisé en ce qu'à l'étape (d) on linéarise les vecteurs à l'aide d'une endonucléase de restriction et on en effectue une dégradation à l'aide d'une exonucléase, pour obtenir un fragment d'ADN.

4. Procédé selon la revendication 3, caractérisé en ce qu'on utilise Bal3L comme exonucléase.

5. Procédé selon l'une des revendications 1 à 4, caractérisé en ce que, dans l'étape (e) on utilise des plasmides promoteurs de tac (comme pKK223-3 et le plasmide pouvant être obtenu à partir de pKK223-3 par séparation d'un fragment BamHI-XmaIII de 546 paires de bases ainsi que par insertion d'un fragment de 1,26 kb contenant le gène répresseur de lactose), des plasmides promoteurs de type Lambda-p_{L} (comme pPL-Lambda) et des plasmides promoteurs trp (comme pDR720).

6. Procédé selon l'une des revendications 1 à 5, caractérisé en ce que, dans l'étape (e), on utilise des fragments d'ADN dont les brins individuels peuvent être hybridés avec les brins individuels des fragments d'ADN découpés lors de l'étape (d), en opérant à au moins 20° C et en particulier à une concentration de NaCl de 1 M et à une température d'au moins 25°C.
